# EUROPEAN PATENT SPECIFICATION

(11) **EP 1 483 394 B1**
(45) Date of publication and mention of the grant of the patent: **15.04.2009**
(21) Application number: 03717198.0
(22) Date of filing: 27.02.2003
(51) Int. Cl.: C12P 13/04, C12P 13/08, C07K 14/245

(54) **PROCESS FOR THE PREPARATION OF L-AMINO ACIDS USING STRAINS OF THE ENTEROBACTERIACEAE FAMILY**
VERFAHREN ZUR HERSTELLUNG VON L-AMINOSÄUREN UNTER VERWENDUNG VON STÄMMEN DER FAMILIE ENTEROBACTERIACEAE
PROCEDE DE PREPARATION D'ACIDES L-AMINES AU MOYEN DE SOUCHES DE LA FAMILLE ENTEROBACTERIACEAE

(30) Priority: 13.03.2002 DE 10210958; 21.03.2002 US 365825 P
(43) Date of publication of application: 08.12.2004
(73) Proprietor: Evonik Degussa GmbH, 45128 Essen (DE)
(72) Inventor: RIEPING, Mechthild, 33619 Bielefeld (DE); HERMANN, Thomas, 33739 Bielefeld (DE); FARWICK, Mike, 45131 Essen (DE)
(86) International application number: PCT/EP2003/001994
(87) International publication number: WO 2003/076636

(56) References cited:
- EP-A- 0 994 190
- WO-A-99/53035
- US-A- 4 278 765
- WATERMAN S R ET AL.: "Identification of sigma S-dependent genes associated with the stationary-phase acid-resistance phenotype of Shigella flexneri." MOLECULAR MICROBIOLOGY, vol. 21, no. 5, September 1996 (1996-09), pages 925-940, XP008020564 ISSN: 0950-382X
- GAJIWALA K S ET AL.: "HDEA, a periplasmic protein that supports acid resistance in pathogenic enteric bacteria." JOURNAL OF MOLECULAR BIOLOGY, vol. 295, no. 3, 21 January 2000 (2000-01-21), pages 605-612, XP002250389 ISSN: 0022-2836 cited in the application
- YOSHIDA T ET AL.: "Function of the Escherichia coli nucleoid protein, H-NS: molecular analysis of a subset of proteins whose expression is enhanced in a hns deletion mutant." MOLECULAR & GENERAL GENETICS, vol. 237, no. 1-2, February 1993 (1993-02), pages 113-122, XP001160777 ISSN: 0026-8925 cited in the application
- YOSHIDA T ET AL.: "Physical map location of a set of Escherichia coli genes (hde) whose expression is affected by the nucleoid protein H-NS." JOURNAL OF BACTERIOLOGY, vol. 175, no. 23, December 1993 (1993-12), pages 7747-7748, XP008020527 ISSN: 0021-9193 cited in the application
- WASINGER V C ET AL.: "Small genes/gene-products in Escherichia coli K-12." FEMS MICROBIOLOGY LETTERS, vol. 169, no. 2, 15 December 1998 (1998-12-15), pages 375-382, XP002250388 ISSN: 0378-1097 cited in the application
- MICHAL G: "Biochemical pathways: an atlas of biochemistry and molecular biology" BIOCHEMICAL PATHWAYS: AN ATLAS OF BIOCHEMISTRY AND MOLECULAR BIOLOGY, 1999, pages 42-54, XP002242199
- KRAEMER R: "Genetic and physiological approaches for the production of amino acids" JOURNAL OF BIOTECHNOLOGY, vol. 45, no. 1, 1996, pages 1-21, XP002178648 ISSN: 0168-1656
- JETTEN M S M ET AL.: "Recent advances in thephysiology and genetics of amino acid-producing bacteria" CRC CRITICAL REVIEWS IN BIOTECHNOLOGY, vol. 15, no. 1, 1995, pages 73-103, XP000613291 ISSN: 0738-8551

## Description

### Field of the Invention

This invention relates to a process for the preparation of L-amino acids, in particular L-threonine, using strains of the Enterobacteriaceae family in which the genes hdeA and hdeB are over-expressed.

### Prior Art

L-amino acids, in particular L-threonine, find application in human medicine and in the pharmaceuticals industry, in the foodstuffs industry and quite particularly in animal nutrition.

It is known that L-amino acids are prepared by fermentation of strains of Enterobacteriaceae, in particular Escherichia coli (E. coli) and Serratia marcescens. Because of their great importance, work is constantly being undertaken to improve the preparation processes. Improvements to the process can relate to technical fermentation measures, such as, for example, stirring and supply of oxygen, or to the composition of the nutrient media, such as, for example, the sugar concentration during fermentation, or to the reworking into product form, for example by ion-exchange chromatography, or to the intrinsic output properties of the microorganism itself.

Methods of mutagenesis, selection and mutant selection are used to improve the output properties of these microorganisms. Strains that are resistant to antimetabolites, such as, for example, the threonine analogue α-amino-β-hydroxyvaleric acid (AHV), or that are auxotrophic in respect of metabolites of regulatory importance and produce L-amino acid, such as, for example, L-threonine, are obtained in this manner.

Methods of recombinant DNA technology have also been employed for some years for strain improvement of strains of the Enterobacteriaceae family that produce L-amino acids, by amplifying individual amino-acid-biosynthesis genes and investigating the effect on production.

### Object of the Invention

The inventors had the object of providing new measures for improved preparation of L-threonine and L-homoserine.

### Summary of the Invention

The invention provides a process for the preparation of said L-amino acids, in particular L-threonine, using microorganisms of the Enterobacteriaceae family which, in particular, already produce L-amino acids and in which the genes hdeA and hdeB are over-expressed.

### Detailed Description of the Invention

Where L-amino acids or amino acids are mentioned in the following, these terms mean one or more amino acids, including their salts, selected from the group comprising L-threonine and L-homoserine. L-threonine is particularly preferred.

The term "enhancement" in this connection describes the increase in the intracellular activity of one or more enzymes or proteins in a microorganism that are coded by the corresponding DNA, for example by increasing the number of copies of the gene or genes, using a strong promoter or a gene or allele that codes for a corresponding enzyme or protein with a high activity, and optionally combining these measures.

By the measures of enhancement, in particular over-expression, the activity or concentration of the corresponding protein is generally increased by at least 10 %, 25 %, 50 %, 75 %, 100 %, 150 %, 200 %, 300%, 400 % or 500 %, up to a maximum of 1000 % or 2000 %, relative to that of the wild-type protein or the activity or concentration of the protein in the initial microorganism.

The process is characterized in that the following steps are carried out:
a) fermentation of microorganisms of the Enterobacteriaceae family which produce the desired L-amino acid and in which the genes hdeA and hdeB are over-expressed,
b) concentration of said L-amino acids in the medium or in the cells of the microorganisms, and
c) isolation of said L-amino acids whereby constituents of the fermentation broth and/or the biomass in its entirety or in proportions ≥ 0 to 100%) thereof optionally remain in the product.

The microorganisms that the present invention provides can produce said L-amino acids from glucose, sucrose, lactose, fructose, maltose, molasses, optionally starch, optionally cellulose or from glycerol and ethanol. They are representatives of the Enterobacteriaceae family selected from the genera Escherichia, Erwinia, Providencia and Serratia. The genera Escherichia and Serratia are preferred. Of the genus Escherichia the species Escherichia coli, and of the genus Serratia the species Serratia marcescens, are to be mentioned in particular.

Suitable strains, which produce L-threonine in particular, of the genus Escherichia, in particular of the species Escherichia coli, are, for example:
Escherichia coli TF427
Escherichia coli H4578
Escherichia coli KY10935
Escherichia coli VNIIgenetika MG442
Escherichia coli VNIIgenetika M1
Escherichia coli VNIIgenetika 472T23
Escherichia coli BKIIM B-3996
Escherichia coli kat 13
Escherichia coli KCCM-10132.

Suitable L-threonine-producing strains of the genus Serratia, in particular of the species Serratia marcescens, are, for example:
Serratia marcescens HNr21
Serratia marcescens TLr156
Serratia marcescens T2000.

Strains from the Enterobacteriaceae family that produce L-threonine preferably have, inter alia, one or more genetic or phenotypic features selected from the group comprising: resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidin, resistance to rifampicin, resistance to valine analogues such as, for example, valine hydroxamate, resistance to purine analogues such as, for example, 6-dimethylaminopurine, a need for L-methionine, optionally a partial and compensable need for L-isoleucine, a need for meso-diaminopimelic acid, auxotrophy in respect of threonine-containing dipeptides, resistance to L-threonine, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally a capacity for sucrose utilization, enhancement of the threonine operon, enhancement of homoserine dehydrogenase I-aspartate kinase I, preferably of the feedback-resistant form, enhancement of homoserine kinase, enhancement of threonine synthase, enhancement of aspartate kinase, optionally of the feedback-resistant form, enhancement of aspartate semialdehyde dehydrogenase, enhancement of phosphoenol pyruvate carboxylase, optionally of the feedback-resistant form, enhancement of phosphoenol pyruvate synthase, enhancement of transhydrogenase, enhancement of the RhtB gene product, enhancement of the RhtC gene product, enhancement of the YfiK gene product, enhancement of a pyruvate carboxylase, and attenuation of acetic-acid formation.

It has been found that microorganisms of the Enterobacteriaceae family produce said L-amino acids, in particular L-threonine, in improved manner after over-expression of the genes hdeA and hdeB.

The nucleotide sequences of the genes of Escherichia coli belong to the prior art (see the following text references) and can also be found in the genome sequence of Escherichia coli published by Blattner et al. (Science 277: 1453-1462 (1997)).
- hdeA gene::
- Description:: Periplasmic protein, chaperonin-like function, imparts resistance to acids
- Reference:: Yoshida et al.; Journal of Bacteriology 175(23): 7747-7748 (1993) Yoshida et al.; Molecular and General Genetics 237(1-2): 113-122 (1993) Wasinger and Humphery-Smith; FEMS Microbiological Letters 169(2): 375-382 (1998)
- Accession No.:: AE000427
- hdeB gene::
- Description:: Periplasmic protein, chaperonin-like function, imparts resistance to acids
- Reference:: Yoshida et al.; Journal of Bacteriology 175(23): 7747-7748 (1993) Yoshida et al.; Molecular and General Genetics 237(1-2): 113-122 (1993) Wasinger and Humphery-Smith; FEMS Microbiological Letters 169(2): 375-382 (1998)
- Accession No.:: AE000427

The nucleic-acid sequences can be found in the databases of the National Center for Biotechnology Information (NCBI) of the National Library of Medicine (Bethesda, MD, USA), in the Nucleotide Sequence Database of the European Molecular Biologies Laboratories (EMBL, Heidelberg, Germany or Cambridge, UK) or in the DNA Database of Japan (DDBJ, Mishima, Japan).

The genes described in the specified text references can be used in accordance with the invention. Alleles of the genes that arise from the degeneracy of the genetic code or as a result of functionally neutral sense mutations can furthermore be used. The use of endogenous genes is preferred.

"Endogenous genes" or "endogenous nucleotide sequences" are to be understood as meaning the genes or alleles or nucleotide sequences that are present in the population of a species.

To achieve an enhancement, the expression of the genes or the catalytic properties of the proteins can, for example, be enhanced. The two measures can optionally be combined.

The identification, determination of concentration and localization of the periplasmic protein for which the polynucleotide hdeA codes are described in Link et al. (Electrophoresis 18(8), 1259-1313 (1997)) and in Gajiwala and Burley (Journal of Molecular Biology 295(3), 605-612 (2000)).

To achieve an over-expression, the number of copies of the corresponding genes can be increased, or the promoter and regulation region or the ribosome-binding site upstream of the structural gene can be mutated. Expression cassettes that are incorporated upstream of the structural gene act in the same way. By means of inducible promoters it is additionally possible to intensify the expression in the course of fermentative production of L-threonine. The expression is likewise improved by measures to prolong the life of the mRNA. Furthermore, the enzyme activity is also increased by preventing the degradation of the enzyme protein. The genes or gene constructs can either be present in plasmids with a varying number of copies or can be integrated and amplified in the chromosome. Alternatively, an over-expression of the genes in question can furthermore be achieved by changing the composition of the media and the culture procedure.

A person skilled in the art can find instructions on this, inter alia, in Chang and Cohen (Journal of Bacteriology 134: 1141-1156 (1978)), in Hartley and Gregori (Gene 13: 347-353 (1981)), in Amann and Brosius (Gene 40: 183-190 (1985)), in de Broer et al. (Proceedings of the National Academy of Sciences of the United States of America 80: 21-25 (1983)), in LaVallie et al. (BIO/TECHNOLOGY 11: 187-193 (1993)), in PCT/US97/13359, in Llosa et al. (Plasmid 26: 222-224 (1991)), in Quandt and Klipp (Gene 80: 161-169 (1989)), / in Hamilton et al. (Journal of Bacteriology 171: 4617-4622 (1989)), in Jensen and Hammer (Biotechnology and Bioengineering 58: 191-195 (1998)) and in known textbooks on genetics and molecular biology.

Plasmid vectors that can replicate in Enterobacteriaceae, such as, for example, cloning vectors derived from pACYC184 (Bartolomé et al.; Gene 102: 75-78 (1991)), pTrc99A (Amann et al.; Gene 69: 301-315 (1988)) or pSC101 derivatives (vocke and Bastia, Proceedings of the National Academy of Sciences USA 80(21): 6557-6561 (1983)) can be used. A strain transformed with a plasmid vector, where the plasmid vector carries at least one of the genes selected from the group comprising hdeA and hdeB, or nucleotide sequences that code for them, can be employed in a process according to the invention.

It is also possible to transfer mutations that affect the expression of the particular genes into various strains by sequence exchange (Hamilton et al.; Journal of Bacteriology 171: 4617-4622 (1989)), conjugation or transduction.

It may furthermore be advantageous for the production of L-threonine with strains of the Enterobacteriaceae family to over-express one or more enzymes of the known threonine-biosynthesis pathway or enzymes of anaplerotic metabolism or enzymes for the production of reduced nicotinamide adenine dinucleotide phosphate or enzymes of glycolysis or PTS enzymes or enzymes of sulfur metabolism, in addition to the over-expression of the genes hdeA and hdeB. The use of endogenous genes is generally preferred.

Thus, for example, at the same time one or more of the genes selected from the group comprising
- the thrABC operon which codes for aspartate kinase, homoserine dehydrogenase, homoserine kinase and threonine synthase (US-A-4,278,765),
- the pyc gene which codes for pyruvate carboxylase (DE-A-19 831 609),
- the pps gene which codes for phosphoenol pyruvate synthase (Molecular and General Genetics 231(2): 332-336 (1992)),
- the ppc gene which codes for phosphoenol pyruvate carboxylase (Gene 31: 279-283 (1984)),
- the pntA and pntB genes which code for transhydrogenase (European Journal of Biochemistry 158: 647-653 (1986)),
- the rhtB gene which imparts homoserine resistance (EP-A-0 994 190),
- the mqo gene which codes for malate:quinone oxidoreductase (DE 100 348 33.5),
- the rhtC gene which imparts threonine resistance (EP-A-1 013 765),
- the thrE gene of Corynebacterium glutamicum which codes for the threonine-export protein (DE 100 264 94.8),
- the gdhA gene which codes for glutamate dehydrogenase (Nucleic Acids Research 11: 5257-5266 (1983); Gene 23: 199-209 (1983)),
- the hns gene which codes for the DNA-binding protein HLP-II (Molecular and General Genetics 212: 199-202 (1988)),
- the pgm gene which codes for phosphoglucomutase (Journal of Bacteriology 176: 5847-5851 (1994)),
- the fba gene which codes for fructose biphosphate aldolase (Biochemical Journal 257: 529-534 (1989)),
- the ptsH gene of the ptsHIcrr operon which codes for phosphohistidine protein hexose phosphotransferase of the phosphotransferase system PTS (Journal of Biological Chemistry 262: 16241-16253 (1987)),
- the ptsI gene of the ptsHIcrr operon which codes for enzyme I of the phosphotransferase system PTS (Journal of Biological Chemistry 262: 16241-16253 (1987)),
- the crr gene of the ptsHIcrr operon which codes for the glucose-specific IIA component of the phosphotransferase system PTS (Journal of Biological Chemistry 262: 16241-16253 (1987)),
- the ptsG gene which codes for the glucose-specific IIBC component (Journal of Biological Chemistry 261: 16398-16403 (1986)),
- the lrp gene which codes for the regulator of the leucine regulon (Journal of Biological Chemistry 266: 10768-10774 (1991)),
- the csrA gene which codes for the global regulator Csr (Journal of Bacteriology 175: 4744-4755 (1993)),
- the fadR gene which codes for the regulator of the fad regulon (Nucleic Acids Research 16: 7995-8009 (1988)),
- the iclR gene which codes for the regulator of central intermediary metabolism (Journal of Bacteriology 172: 2642-2649 (1990)),
- the mopB gene which codes for the 10 Kd chaperone (Journal of Biological Chemistry 261: 12414-12419 (1986)) and is also known by the name 'groES',
- the ahpC gene of the ahpCF operon which codes for the small subunit of alkyl hydroperoxide reductase (Proceedings of the National Academy of Sciences USA 92: 7617-7621 (1995)),
- the ahpF gene of the ahpCF operon which codes for the large subunit of alkyl hydroperoxide reductase (Proceedings of the National Academy of Sciences USA 92: 7617-7621 (1995)),
- the cysK gene which codes for cysteine synthase A (Journal of Bacteriology 170: 3150-3157 (1988)),
- the cysB gene which codes for the regulator of the cys regulon (Journal of Biological Chemistry 262: 5999-6005 (1987)),
- the cysJ gene of the cysJIH operon which codes for the flavoprotein of NADPH sulfite reductase (Journal of Biological Chemistry 264: 15796-15808 (1989), Journal of Biological Chemistry 264: 15726-15737 (1989)),
- the cysI gene of the cysJIH operon which codes for the haemoprotein of NADPH sulfite reductase (Journal of Biological Chemistry 264: 15796-15808 (1989), Journal of Biological Chemistry 264: 15726-15737 (1989)),
- the cysH gene of the cysJIH operon which codes for adenylyl sulfate reductase (Journal of Biological Chemistry 264: 15796-15808 (1989), Journal of Biological Chemistry 264: 15726-15737 (1989)),
- the phoB gene of the phoBR operon which codes for the positive regulator PhoB of the pho regulon (Journal of Molecular Chemistry 190 (1): 37-44 (1986)),
- the phoR gene of the phoBR operon which codes for the sensor protein of the pho regulon (Journal of Molecular Biology 192 (3): 549-556 (1986)),
- the phoE gene which codes for protein E of the outer cell membrane (Journal of Molecular Biology 163 (4): 513-532 (1983)),
- the pykF gene which codes for pyruvate kinase I which is stimulated by fructose (Journal of Bacteriology 177 (19): 5719-5722 (1995)),
- the pkfB gene which codes for 6-phosphofructokinase II (Gene 28 (3): 337-342 (1984)),
- the malE gene which codes for the periplasmic binding protein of maltose transport (Journal of Biological Chemistry 259 (16): 10606-10613 (1984)),
- the rseA gene of the rseABC operon which codes for a membrane protein with anti-sigmaE activity (Molecular Microbiology 24 (2): 355-371 (1997)),
- the rseC gene of the rseABC operon which codes for a global regulator of the sigmaE factor (Molecular Microbiology 24 (2): 355-371 (1997)),
- the sodA gene which codes for superoxide dismutase (Journal of Bacteriology 155 (3): 1078-1087 (1983)),
- the sucA gene of the sucABCD operon which codes for the decarboxylase subunit of 2-ketoglutarate dehydrogenase (European Journal of Biochemistry 141 (2): 351-359 (1984)),
- the sucB gene of the sucABCD operon which codes for the dihydrolipoyl transsuccinase E2 subunit of 2-ketoglutarate dehydrogenase (European Journal of Biochemistry 141 (2): 361-374 (1984)),
- the sucC gene of the sucABC operon which codes for the β-subunit of succinyl-CoA synthetase (Biochemistry 24 (22): 6245-6252 (1985)) and
- the sucD gene of the sucABCD operon which codes for the α-subunit of succinyl-CoA synthetase (Biochemistry 24 (22): 6245-6252 (1985))
can be over-expressed.

It may furthermore be advantageous for the production of L-threonine, in addition to the over-expression of the genes hdeA and hdeB, for one or more genes selected from the group comprising
- the tdh gene which codes for threonine dehydrogenase (Journal of Bacteriology 169: 4716-4721 (1987)),
- the mdh gene which codes for malate dehydrogenase (E.C. 1.1.1.37) (Archives in Microbiology 149: 36-42 (1987)),
- the gene product of the open reading frame (orf) yjfA (Accession Number AAC77180 of the National Center for Biotechnology Information (NCBI, Bethesda, MD, USA)),
- the gene product of the open reading frame (orf) ytfP (Accession Number AAC77179 of the National Center for Biotechnology Information (NCBI, Bethesda, MD, USA)),
- the pckA gene which codes for the enzyme phosphoenol pyruvate carboxykinase (Journal of Bacteriology 172: 7151-7156 (1990)),
- the poxB gene which codes for pyruvate oxidase (Nucleic Acids Research 14 (13): 5449-5460 (1986)),
- the aceA gene which codes for the enzyme isocitrate lyase (Journal of Bacteriology 170: 4528-4536 (1988)),
- the dgsA gene which codes for the DgsA regulator of the phosphotransferase system (Bioscience, Biotechnology and Biochemistry 59: 256-261 (1995)) and is also known by the name 'mlc gene',
- the fruR gene which codes for the fructose repressor (Molecular and General Genetics 226: 332-336 (1991)) and is also known by the name 'cra gene',
- the rpoS gene which codes for the sigma³⁸ factor (WO 01/05939) and is also known by the name 'katF gene',
- the aspA gene which codes for aspartate ammonium lyase, (aspartase) (Nucleic Acids Research 13(6): 2063-2074 (1985)) and
- the aceB gene which codes for malate synthase A (Nucleic Acids Research 16(19): 9342 (1988))
to be eliminated.

The term "attenuation" in this connection describes the reduction or elimination of the intracellular activity of one or more enzymes (proteins) in a microorganism that are coded by the corresponding DNA, for example by using a weak promoter or a gene or allele that codes for a corresponding enzyme with a low activity or inactivates the corresponding enzyme (protein) or gene, and optionally combining these measures.

By the measures of attenuation, the activity or concentration of the corresponding protein is generally lowered to 0 to 75 %, 0 to 50 %, 0 to 25 %, 0 to 10 % or 0 to 5% of the activity or concentration of the wild-type protein, or of the activity or concentration of the protein in the initial microorganism.

It may furthermore be advantageous for the production of said L-amino acids, in particular L-threonine, in addition to over-expression of the genes hdeA and hdeB, to eliminate undesirable side reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms produced in accordance with the invention can be cultured in the batch process (batch cultivation), in the fed-batch process or in the repeated-fed-batch process. A summary of known cultivation methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must satisfy the requirements of the particular strains in suitable manner. Descriptions of culture media for various microorganisms are contained in the handbook "Manual of Methods for General Bacteriology" of the American Society for Bacteriology (Washington D.C., USA, 1981).

Sugars and carbohydrates, such as, for example, glucose, sucrose, lactose, fructose, maltose, molasses, starch and optionally cellulose, oils and fats, such as, for example, soybean oil, sunflower oil, peanut oil and coconut oil, fatty acids, such as, for example, palmitic acid, stearic acid and linoleic acid, alcohols, such as, for example, glycerol and ethanol, and organic acids, such as, for example, acetic acid, can be used as the source of carbon. These substances can be used individually or as a mixture.

Organic nitrogenous compounds, such as peptones, yeast extract, meat extract, malt extract, corn steep liquor, soybean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogenphosphate or dipotassium hydrogenphosphate or the corresponding sodium-containing salts can be used as the source of phosphorus. The culture medium must furthermore contain salts of metals, such as, for example, magnesium sulfate or iron sulfate, which are necessary for growth. Finally, essential growth substances, such as amino acids and vitamins, can be employed in addition to the above-mentioned substances. Suitable precursors can moreover be added to the culture medium. The named feed substances can be added to the culture in the form of a single charge or can be fed in during culturing in suitable manner.

The fermentation is generally carried out at a pH of 5.5 to 9.0, in particular 6.0 to 8.0. Basic compounds, such as sodium hydroxide, potassium hydroxide, ammonia or aqueous ammonia, or acidic compounds, such as phosphoric acid or sulfuric acid, can be employed in suitable manner to control the pH of the culture. Anti-foaming agents, such as, for example, fatty-acid polyglycol esters, can be employed to control the evolution of foam. Suitable substances having a selective action, for example antibiotics, can be added to the medium to maintain the stability of plasmids. To maintain aerobic conditions, oxygen or oxygenous gas mixtures, such as air for example, are introduced into the culture. The temperature of the culture is usually 25°C to 45°C, and preferably 30°C to 40°C. Culturing is continued until a maximum of L-amino acids or L-threonine has formed. This target is usually reached within 10 hours to 160 hours.

The analysis of L-amino acids can be undertaken by anion-exchange chromatography with subsequent ninhydrin derivation, as described by Spackman et al. (Analytical Chemistry, 30: 1190-1206 (1958)), or it can be undertaken by reversed-phase HPLC, as described by Lindroth et al. (Analytical Chemistry 51: 1167-1174 (1979)).

The process according to the invention serves for the fermentative preparation of L-amino acids such as, for example, L-threonine and L-homoserine, in particular L-threonine.

The present invention will be elucidated in more detail in the following on the basis of exemplary embodiments.

Minimal media (M9) and complete media (LB) used for Escherichia coli are described by J.H. Miller (A Short Course in Bacterial Genetics (1992), Cold Spring Harbor Laboratory Press). The isolation of plasmid DNA from Escherichia Coli, and also all the techniques for the restriction, ligation, Klenow treatment and alkaline phosphatase treatment are carried out in accordance with Sambrook et al. (Molecular Cloning - A Laboratory Manual (1989), Cold Spring Harbor Laboratory Press). The transformation of Escherichia coli is carried out, unless otherwise described, in accordance with Chung et al. (Proceedings of the National Academy of Sciences of the United States of America (1989) 86: 2172-2175).

The incubation-temperature in the preparation of strains and transformants is 37 °C.

### Example 1

### Construction of the expression plasmid pTrc99AhdeAB

The hdeAB gene region from E. coli K12 is amplified by applying the polymerase chain reaction (PCR) and also synthetic oligonucleotides. Proceeding from the nucleotide sequence of the hdeAB gene region in E. coli K12 MG1655 (Accession Number AE000427, Blattner et al. (Science 277(5331): 1453-1474 (1997)), PCR primers are synthesized (MWG Biotech, Ebersberg, Germany). The sequences of the primers are modified in such a way that recognition sites for restriction enzymes arise. For the hdeAB1 primer the recognition sequence for XbaI is chosen, and for the hdeAB2 primer the recognition sequence for HindIII is chosen, these recognition sequences being marked by underlining in the nucleotide sequences represented below:
hdeAB1:
   5' -GACGGCTCTTTCTCTAGATAGTTGAGG- 3' (SEQ ID No. 1)
hdeAB2:
   5' -GCAAGATGGCTAAGCTTGCTACTCC- 3' (SEQ ID No. 2)

The chromosomal E. coli K12 MG1655 DNA employed for the PCR is isolated, in accordance with the manufacturer's instructions, with "Qiagen Genomic-tips 100/G" (QIAGEN, Hilden, Germany). A DNA fragment having a size of about 850 bp can be amplified with the specific primers under standard PCR conditions (Innis et al. (1990) PCR Protocols. A Guide to Methods and Applications, Academic Press) with Vent-Polymerase (New England Biolabs GmbH, Frankfurt, Germany). The amplified DNA fragment is identified via gel electrophoresis in an agarose gel (0.8 %) and, according to the manufacturer's instructions, is employed for ligation with the vector pCR-Blunt II-TOPO (Zero Blunt TOPO PCR Cloning Kit, Invitrogen, Groningen, Netherlands).
Following this, transformation into the E. coli strain TOP10 (Invitrogen, Groningen, Netherlands) is undertaken. The selection of plasmid-carrying cells is undertaken on LB agar which is pricked with 50 mg/l kanamycin. After the isolation of plasmid DNA, the vector is examined by means of restriction cleavage with the restriction enzymes Xba1 and HindIII (Invitrogen, Groningen, Netherlands) and is identified in the agarose gel (0.8 %). The plasmid that is obtained is designated by pCRBlunthdeAB.

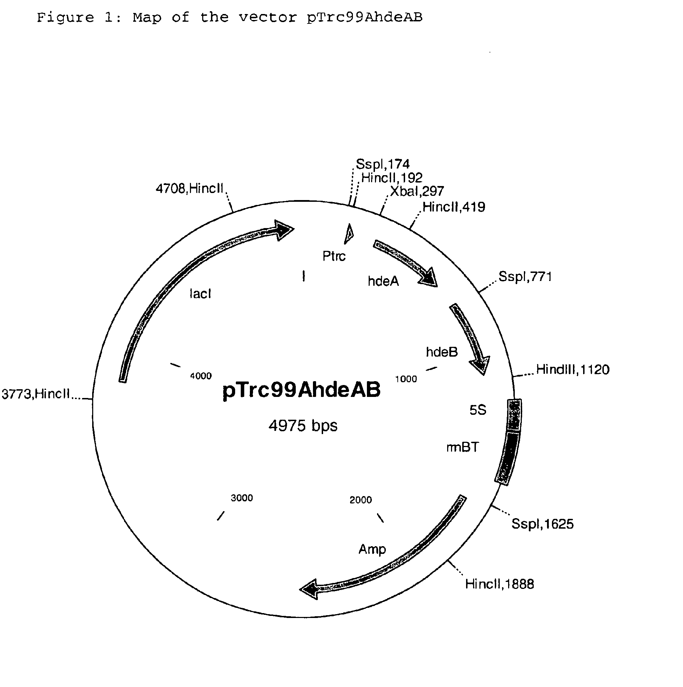

The plasmid pCRBlunthdeAB that has been described is cut with the restriction enzymes Xba1 and HindIII. After fractionation in an agarose gel (0.8%), an hdeAB fragment having a size of about 0.8 kbp is isolated from the agarose gel with the aid of the High Pure Product Purification Kit (Roche Diagnostics GmbH, Mannheim, Germany) and is employed for ligation with the vector pTrc99A (Pharmacia Biotech, Uppsala, Sweden). Said vector is previously likewise cleaved with the restriction enzymes Xba1 and HindIII, mixed with the isolated hdeAB fragment and treated with T4-DNA-Ligase (Amersham-Pharmacia, Freiburg, Germany). Subsequently the E. coli strain TOP10 (Invitrogen, Groningen, Netherlands) is transformed with the ligation charge, and plasmid-carrying cells are selected on LB agar which is pricked with 50 µg/ml ampicillin. The successful cloning can be confirmed after the isolation of plasmid DNA by control cleavage with the enzymes HincII and SspI. The plasmid is designated as pTrc99AhdeAB (Figure 1).

### Example 2

### Preparation of L-threonine with the strain MG442/pTrc99AhdeAB

The E. coli strain MG442 which produces L-threonine is described in Patent US-A-4,278,765 and has been deposited in the Russian National Collection of Industrial Microorganisms (VKPM, Moscow, Russia) as CMIM B-1628.

The strain MG442 is transformed with the expression plasmid pTrc99AhdeAB described in Example 1 and with the vector pTrc99A and selected in respect of plasmid-carrying cells on LB agar with 50 µg/ml ampicillin. In this way the strains MG442/pTrc99AhdeAB and MG442/pTrc99A arise. Selected single colonies are subsequently multiplied further on minimal medium having the following composition: 3.5 g/l Na₂HPO₄*2H₂O, 1.5 g/l KH₂PO₄, 1 g/l NH₄Cl, 0.1 g/l MgSO₄*7H₂O, 2 g/l glucose, 20 g/l agar, 50 mg/l ampicillin. The formation of L-threonine is examined in batch cultures of 10 ml which are contained in 100 ml Erlenmeyer flasks. To this end, 10 ml of preculture medium having the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose, 50 mg/l ampicillin, is inoculated and incubated for 16 hours at 37°C and at 180 rpm in an ESR incubator manufactured by Kühner AG (Birsfelden, Switzerland). 250 µl at a time of this preculture are inoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃, 20 g/l glucose, 50 mg/l ampicillin) and incubated for 48 hours at 37 °C. With a view to complete induction of the expression of the genes hdeA and hdeB, 100 mg/l isopropyl-β-D-thiogalactopyranoside (IPTG) are added in parallel charges. The formation of L-threonine by the parent strain MG442 is examined in the same way, but without addition of ampicillin to the medium. After the incubation, the optical density (OD) of the culture suspension is determined with an LP2W-Photometer manufactured by Dr. Lange (Düsseldorf, Germany) at a measuring-wavelength of 660 nm.

Subsequently the concentration of L-threonine formed in the sterile-filtered culture supernatant is determined with an amino-acid analyzer manufactured by Eppendorf-BioTronik (Hamburg, Germany) by ion-exchange chromatography and post-column reaction with detection of ninhydrin.

The result of the experiment is presented in Table 1.

**Table 1**

| Strain | Admixtures | OD (660 nm) | L-threonine g/l |
|---|---|---|---|
| MG442 | - | 5.6 | 1.4 |
| MG442/pTrc99A | - | 3.8 | 1.3 |
| MG442/pTrc99AhdeAB | - | 3.7 | 1.6 |
| MG442/pTrc99AhdeAB | IPTG | 6.7 | 2.2 |

### Brief description of the Figure:

- Figure 1:Map of the plasmid pTrc99AhdeAB containing the hdeAB gene region

Length data are to be interpreted as approximate data. The abbreviations and designations that are used have the following meanings:
- Amp: ampicillin-resistance gene
- lacI: gene for the repressor protein of the trc promoter
- Ptrc: trc promoter region, IPTG-inducible
- hdeA: coding region of the hdeA gene
- hdeB: coding region of the hdeB gene
- 5S: 5S rRNA region
- rrnBT: rRNA terminator region

The abbreviations for the restriction enzymes have the following meanings:
- XbaI: restriction endonuclease derived from *Xanthomonas campestris*
- HindIII: restriction endonuclease derived from *Haemophilus influenzae R_{c}*
- HincII: restriction endonuclease derived from *Haemophilus influenzae R_{d}*
- SspI: restriction endonuclease derived from *Sphaerotilus* sp. ATTC® 13925

## Claims

1. A process for the preparation of L-threonine or L-homoserine, wherein the following steps are carried out:
a) fermentation of modified microorganisms of the Enterobacteriaceae family in which the genes hdeA and hdeB, which code for periplasmic proteins supporting acid resistance, are over-expressed,
b) accumulation of the desired L-amino acid in the medium or in the cells of the microorganisms, and
c) isolation of the desired L-amino acid, whereby constituents of the fermentation broth and/or the biomass in its entirety or portions (> 0 to 100%) thereof optionally remain in the product.

2. Process according to claim 1, wherein the polynucleotides of the genes hdeA and hdeB are over-expressed by an inducible promoter.

3. Process according to claim 1, wherein for the preparation of L-threonine microorganisms of the Enterobacteriaceae family are fermented in which additionally at the same time one or more of the genes selected from the group comprising:
3.1 the thrABC operon which codes for aspartate kinase, homoserine dehydrogenase, homoserine kinase and threonine synthase,
3.2 the pyc gene which codes for pyruvate carboxylase,
3.3 the pps gene which codes for phosphoenolpyruvate synthase,
3.4 the ppc gene which codes for phosphoenolpyruvate carboxylase,
3.5 the pntA and pntB genes which code for transhydrogenase,
3.6 the Escherichia coli rhtB gene coding for a protein imparting homoserine resistance,
3.7 the mqo gene which codes for malate:quinone oxidoreductase,
3.8 the Escherichia coli rhtC gene coding for a protein imparting threonine resistance, .
3.9 the thrE gene which codes for the threonine export protein,
3.10 the gdhA gene which codes for glutamate dehydrogenase,
3.11 the hns gene which codes for the DNA-binding protein HLP-II,
3.12 the pgm gene, which codes for phosphoglucoisomerase,
3.13 the fba gene which codes for fructose bisphosphate aldolase,
3.14 the ptsH gene which codes for the phosphohistidine protein hexose phosphotransferase,
3.15 the ptsI gene which codes for enzyme I of the phosphotransferase system,
3.16 the crr gene which codes for the glucose-specific IIA component,
3.17 the ptsG gene which codes for the glucose-specific IIBC component,
3.18 the lrp gene, which codes for the regulator of the leucine regulon,
3.19 the csrA gene, which codes for the global regulator Csr,
3.20 the fadR gene, which codes for the regulator FadR of fatty acid and acetate metabolism,
3.21 the iclR gene, which codes for the regulator IclR,
3.22 the mopB gene, which codes for the 10 kD chaperone,
3.23 the ahpC gene, which codes for the small subunit of alkyl hydroperoxide reductase,
3.24 the ahpF gene, which codes for the large subunit of .. alkyl hydroperoxide reductase,
3.25 the cysK gene, which codes for cysteine synthase A,
3.26 the cysB gene, which codes for the regulator of the cys regulon,
3.27 the cysJ gene, which codes for the flavoprotein of NADPH-sulfite reductase,
3.28 the cysI gene, which codes for the haemoprotein of NADPH-sulfite reductase,
3.29 the cysH gene, which codes for adenylyl sulfate reductase,
3.30 the phoB gene, which codes for the positive regulator PhoB of the pho regulon,
3.31 the phoR gene, which codes for the sensor protein of the pho regulon,
3.32 the phoE gene, which codes for protein E of the outer cell membrane,
3.33 the male gene, which codes for the periplasmatic binding protein of maltose transport,
3.34 the pykF gene, which codes for fructose-stimulated pyruvate kinase I,
3.35 the pfkB gene, which codes for 6-phosphofructokinase II,
3.36 the rseA gene, which codes for a membrane protein which acts as a negative regulator on sigmaE activity,
3.37 the rseC gene, which codes for a global regulator of the sigmaE factor,
3.38 the sodA gene, which codes for superoxide dismutase,
3.39 the sucA gene, which codes for the decarboxylase subunit of 2-ketoglutarate dehydrogenase,
3.40 the sucB gene, which codes for the dihydrolipoyltranssuccinase E2 subunit of 2-ketoglutarate dehydrogenase,
3.41 the sucC gene, which codes for the β-subunit of succinyl-CoA synthetase and
3.42 the sucD gene, which codes for the α-subunit of succinyl-CoA synthetase
is or are over-expressed.

4. Process according to claim 1, wherein for the preparation of L-threonine microorganisms of the Enterobacteriaceae family are fermented in which additionally at the same time one or more of the genes selected from the group comprising:
4.1 the tdh gene which codes for threonine dehydrogenase,
4.2 the mdh gene which codes for malate dehydrogenase,
4.3 the gene product of the open reading frame (orf) yjfA of E. coli, when said microorganism is Escherichia coli,
4.4 the gene product of the open reading frame (orf) ytfP of E. coli, when said microorganism is Escherichia coli,
4.5 the pckA gene which codes for phosphoenolpyruvate carboxykinase,
4.6 the poxB gene which codes for pyruvate oxidase,
4.7 the aceA gene which codes for isocitrate lyase,
4.8 the dgsA gene which codes for the DgsA regulator of the phosphotransferase system,
4.9 the fruR gene which codes for the fructose repressor,
4.10 the rpoS gene which codes for the sigma³⁸ factor,
4.11 the aspA gene which codes for the aspartate ammonia-lyase (aspartase) and
4.12 the aceB gene which codes for malate synthase A'
is or are eliminated.

5. Recombinant microorganisms of the Enterobacteriaceae family, which produce L-threonine and wherein at least:
a) the hdeA and hdeB genes, coding for periplasmic proteins supporting acid resistance, or nucleotide sequences which code for the hdeA and hdeB gene products, and
b) the thrABC operon which genes code for aspartate kinase, homoserine dehydrogenase, homoserine kinase and threonine synthase,
are overexpressed.

6. Microorganisms according to claim 5, wherein the microorganisms originate from the species E. coli.

## Patentansprüche

1. Verfahren zur Herstellung von L-Threonin oder L-Homoserin, bei dem man folgende Schritte durchführt:
a) Fermentation von modifizierten Mikroorganismen der Familie Enterobacteriaceae, in denen die Gene hdeA und hdeB, die für die Säureresistenz unterstützende periplasmatische Proteine codieren, überexprimiert sind,
b) Anhäufung der gewünschten L-Aminosäure im Medium oder in den Zellen der Mikroorganismen und
c) Isolierung der gewünschten L-Aminosäure, wodurch gegebenenfalls Bestandteile der Fermentationsbrühe und/oder die Biomasse in ihrer Gesamtheit oder Anteilen (> 0 bis 100%) davon im Produkt verbleiben.

2. Verfahren nach Anspruch 1, wobei die Polynukleotide der Gene hdeA und hdeB über einen induzierbaren Promotor überexprimiert werden.

3. Verfahren nach Anspruch 1, bei dem man zur Herstellung von L-Threonin Mikroorganismen der Familie Enterobacteriaceae fermentiert, in denen man zusätzlich gleichzeitig eines oder mehrere der Gene, ausgewählt aus der Gruppe:
3.1 das für die Aspartatkinase, die Homoserin-Dehydrogenase, die Homoserinkinase und die Threoninsynthase kodierende thrABC-Operon,
3.2 das für die Pyruvat-Carboxylase kodierende pyc-Gen,
3.3 das für die Phosphoenolpyruvat-Synthase kodierende pps-Gen,
3.4 das für die Phosphoenolpyruvat-Carboxylase kodierende ppc-Gen,
3.5 die für die Transhydrogenase kodierenden Gene pntA und pntB,
3.6 das für ein Homoserinresistenz vermittelndes Protein kodierende Gen rhtB von Escherichia coli,
3.7 das für die Malat:Chinon Oxidoreduktase kodierende mqo-Gen,
3.8 das für ein Threoninresistenz vermittelndes Protein kodierende Gen rhtC von Escherichia coli,
3.9 das für das Threoninexportprotein kodierende thrE-Gen,
3.10 das für die Glutamat-Dehydrogenase kodierende gdhA-Gen,
3.11 das für das DNA-Bindeprotein HLP-II kodierende hns-Gen,
3.12 das für die Phosphoglukoisomerase kodierende pgm-Gen,
3.13 das für die Fructose-Bisphosphataldolase kodierende fba-Gen,
3.14 das für die Phosphohistidinproteinhexosephosphotransferase kodierende ptsH-Gen,
3.15 das für Enzym I des Phosphotransferase-Systems kodierende ptsI-Gen,
3.16 das für die glukosespezifische IIA-Komponente kodierende crr-Gen,
3.17 das für die glukosespezifische IIBC Komponente kodierende ptsG-Gen,
3.18 das für den Regulator des Leucin-Regulons kodierende lrp-Gen,
3.19 das für den globalen Regulator Csr kodierende csrA-Gen,
3.20 das für den Regulator FadR des Fettsäure- und Acetatstoffwechsels kodierende fadR-Gen,
3.21 das für den Regulator IclR kodierende iclR-Gen,
3.22 das für das für das Chaperon 10 kD kodierende mopB-Gen,
3.23 das für die kleine Untereinheit der Alkylhydroperoxidreduktase kodierende ahpC-Gen,
3.24 das für die große Untereinheit der Alkylhydroperoxidreduktase kodierende ahpF-Gen,
3.25 das für die Cystein-Synthase A kodierende cysK-Gen,
3.26 das für den Regulator des cys-Regulons kodierende cysB-Gen,
3.27 das für das Flavoprotein der NADPH-Sulfitreduktase kodierende cysJ-Gen,
3.28 das für das Hämoprotein der NADPH-Sulfitreduktase kodierende cysI-Gen,
3.29 das für die Adenylylsulfat-Reduktase kodierende cysH-Gen,
3.30 das für den positiven Regulator PhoB des pho Regulons kodierende phoB-Gen,
3.31 das für das Sensorprotein des pho Regulons kodierende phoR-Gen,
3.32 das für das Protein E der äußeren Zellmembran kodierende phoE-Gen,
3.33 das für das periplasmatische Bindungsprotein des Maltosetransports kodierende malE-Gen,
3.34 das für die fructosestimulierte Pyruvatkinase I kodierende pykF-Gen,
3.35 das für die 6-Phosphofructokinase II kodierende pfkB-Gen,
3.36 das für ein Membranprotein, das als negativer Regulator auf die sigmaE-Aktivität wirkt, kodierende rseA-Gen
3.37 das für einen globalen Regulator des sigmaE-Faktors kodierende rseC-Gen,
3.38 das für die Superoxid-Dismutase kodierende sodA-Gen,
3.39 das für die Decarboxylase-Untereinheit von 2-Ketoglutarat-Dehydrogenase kodierende sucA-Gen,
3.40 das für die Dihydrolipolytranssuccinase E2-Untereinheit von 2-Ketoglutarat-Dehydrogenase kodierende sucB-Gen,
3.41 das für die β-Untereinheit von Succinyl-CoA-Synthetase kodierende sucC-Gen,
3.42 das für die α-Untereinheit von Succinyl-CoA-Synthetase kodierende sucD-Gen,
überexprimiert.

4. Verfahren nach Anspruch 1, bei dem man zur Herstellung von L-Threonin Mikroorganismen der Familie Enterobacteriaceae fermentiert, in denen man zusätzlich gleichzeitig eines oder mehrere der Gene, ausgewählt aus der Gruppe:
4.1 das für die Threonin-Dehydrogenase kodierende tdh-Gen,
4.2 das für die Malat-Dehydrogenase kodierende mdh-Gen,
4.3 falls der genannte Mikroorganismus aus der Spezies E. coli ist, das Genprodukt des offenen Leserahmens (orf) yjfA von Escherichia coli,
4.4 falls der genannte Mikroorganismus aus der Spezies E. coli ist, das Genprodukt des offenen Leserahmens (orf) ytfP von Escherichia coli,
4.5 das für die Phosphoenolpyruvat-Carboxykinase kodierende pckA-Gen,
4.6 das für die Pyruvat-Oxidase kodierende poxB-Gen,
4.7 das für die Isocitrat-Lyase kodierende aceA-Gen,
4.8 das für den DgsA-Regulator des Phosphotransferase-Systems kodierende dgsA-Gen,
4.9 das für den Fructose-Repressor kodierende fruR-Gen,
4.10 das für den Faktor Sigma³⁸ kodierende rpoS-Gen,
4.11 das für die Aspartat-Ammoniak-Lyase (Aspartase) kodierende aspA-Gen und
4.12 das für die Malatsynthase A kodierende aceB-Gen
ausschaltet.

5. Rekombinante Mikroorganismen der Familie Enterobacteriaceae, die L-Threonin produzieren und bei denen wenigstens
a) die für die Säureresistenz unterstützende periplasmatische Proteine kodierenden Gene hdeA und hdeB oder für die Genprodukte von hdeA und hdeB kodierende Nukleotidsequenzen sowie
b) das thrABC-Operon, dessen Gene für die Aspartatkinase, die Homoserin-Dehydrogenase, die Homoserinkinase und die Threoninsynthase kodieren,
überexprimiert sind.

6. Mikroorganismen nach Anspruch 5, bei denen die Mikroorganismen aus der Spezies E. coli sind.

## Revendications

1. Procédé de préparation de L-thréonine ou de L-homosérine, selon lequel les étapes suivantes sont réalisées :
a) la fermentation de microorganismes modifiés de la famille des Enterobacteriaceae dans lesquels les gènes hdeA et hdeB, qui codent pour des protéines périplasmiques supportant la résistance aux acides, sont surexprimés,
b) l'accumulation de l'acide L-aminé souhaité dans le milieu ou dans les cellules des microorganismes, et
c) l'isolement de l'acide L-aminé souhaité, les constituants du bouillon de fermentation et/ou la biomasse en totalité ou en partie (> 0 à 100 %) restant éventuellement dans le produit.

2. Procédé selon la revendication 1, selon lequel les polynucléotides des gènes hdeA et hdeB sont surexprimés par un promoteur inductible.

3. Procédé selon la revendication 1, selon lequel, pour la préparation de L-thréonine, des microorganismes de la famille des Enterobacteriaceae sont fermentés, microorganismes dans lesquels, en outre, en même temps, un ou plusieurs des gènes choisis dans le groupe constitué par :
3.1 l'opéron thrABC qui code pour l'aspartate kinase, l'homosérine déshydrogénase, l'homosérine kinase et la thréonine synthase,
3.2 le gène pyc qui code pour la pyruvate carboxylase,
3.3 le gène pps qui code pour la phosphoénolpyruvate synthase,
3.4 le gène ppc qui code pour la phosphoénolpyruvate carboxylase,
3.5 les gènes pntA et pntB qui codent pour la transhydrogénase,
3.6 le gène rhtB d'Escherichia coli codant pour une protéine qui confère une résistance à l'homosérine,
3.7 le gène mqo qui code pour la malate:quinone oxydoréductase,
3.8 le gène rhtC d'Escherichia coli codant pour une protéine qui confère une résistance à la thréonine,
3.9 le gène thrE qui code pour la protéine d'exportation de la thréonine,
3.10 le gène gdhA qui code pour la glutamate déshydrogénase,
3.11 le gène hns qui code pour la protéine HLP-II se liant à l'ADN,
3.12 le gène pgm qui code pour la phosphoglucoisomérase,
3.13 le gène fba qui code pour la fructose bisphosphate aldolase,
3.14 le gène ptsH qui code pour la phosphohistidine protéine hexose phosphotransférase,
3.15 le gène ptsI qui code pour l'enzyme I du système phosphotransférase,
3.16 le gène crr qui code pour le composant IIA spécifique du glucose,
3.17 le gène ptsG qui code pour le composant IIBC spécifique du glucose,
3.18 le gène lrp qui code pour le régulateur du régulon de la leucine,
3.19 le gène csrA qui code pour le régulateur global Csr,
3.20 le gène fadR qui code pour le régulateur FadR du métabolisme des acides gras et de l'acétate,
3.21 le gène iclR qui code pour le régulateur IclR,
3.22 le gène mopB qui code pour la protéine chaperonne de 10 kD,
3.23 le gène ahpC qui code pour la petite sous-unité de l'alkyl hydroperoxyde réductase,
3.24 le gène ahpF qui code pour la grande sous-unité de l'alkyl hydroperoxyde réductase,
3.25 le gène cysK qui code pour la cystéine synthase A,
3.26 le gène cysB qui code pour le régulateur du régulon cys,
3.27 le gène cysJ qui code pour la flavoprotéine de la NADPH sulfite réductase,
3.28 le gène cysI qui code pour l'hémoprotéine de la NADPH sulfite réductase,
3.29 le gène cysH qui code pour l'adénylylsulfate réductase,
3.30 le gène phoB qui code pour le régulateur positif PhoB du régulon pho,
3.31 le gène phoR qui code pour la protéine capteur du régulon pho,
3.32 le gène phoE qui code pour la protéine E de la membrane cellulaire externe,
3.33 le gène malE qui code pour la protéine de liaison périplasmique du transport du maltose,
3.34 le gène pykF qui code pour la pyruvate kinase I stimulée par le fructose,
3.35 le gène pfkB qui code pour la 6-phosphofructokinase II,
3.36 le gène rseA qui code pour une protéine membranaire qui joue le rôle de régulateur négatif sur l'activité de sigmaE,
3.37 le gène rseC qui code pour un régulateur global du facteur sigmaE,
3.38 le gène sodA qui code pour la superoxyde dismutase,
3.39 le gène sucA qui code pour la sous-unité décarboxylase de la 2-cétoglutarate déshydrogénase,
3.40 le gène sucB qui code pour la sous-unité dihydrolipoyltranssuccinase E2 de la 2-cétoglutarate déshydrogénase,
3.41 le gène sucC qui code pour la sous-unité β de la succinyl-CoA synthétase, et
3.42 le gène sucD qui code pour la sous-unité α de la succinyl-CoA synthétase
est ou sont surexprimés.

4. Procédé selon la revendication 1, selon lequel, pour la préparation de L-thréonine, des microorganismes de la famille des Enterobacteriaceae sont fermentés, microorganismes dans lesquels, en outre, en même temps, un ou plusieurs des gènes choisis dans le groupe constitué par :
4.1 le gène tdh qui code pour la thréonine déshydrogénase,
4.2 le gène mdh qui code pour la malate déshydrogénase,
4.3 le produit génique du cadre ouvert de lecture (orf) yjfA d'E. coli, lorsque ledit microorganisme est Escherichia coli,
4.4 le produit génique du cadre ouvert de lecture (orf) ytfP d'E. coli, lorsque ledit microorganisme est Escherichia coli,
4.5 le gène pckA qui code pour la phosphoénolpyruvate carboxykinase,
4.6 le gène poxB qui code pour la pyruvate oxydase,
4.7 le gène aceA qui code pour l'isocitrate lyase,
4.8 le gène dgsA qui code pour le régulateur DgsA du système phosphotransférase,
4.9 le gène fruR qui code pour le répresseur du fructose, et
4.10 le gène rpoS qui code pour le facteur sigma³⁸,
4.11 le gène aspA qui code pour l'aspartate ammonia-lyase (aspartase), et
4.12 le gène aceB qui code pour la malate synthase A
est ou sont éliminés.

5. Microorganismes recombinants de la famille des Enterobacteriaceae, qui produisent de la L-thréonine et dans lesquels au moins:
a) les gènes hdeA et hdeB, codant pour des protéines périplasmiques supportant la résistance aux acides, ou les séquences nucléotidiques qui codent pour les produits des gènes hdeA et hdeB, et
b) l'opéron thrABC dont les gènes codent pour l'aspartate kinase, l'homosérine déshydrogénase, l'homosérine kinase et la thréonine synthase,
sont surexprimés.

6. Microorganismes selon la revendication 5, les microorganismes provenant de l'espèce E. coli.


### SEQUENCE LISTING

<110> Degussa AG
<120> Process for the preparation of L-amino acids using strains of the Enterobacteriaceae family
<130> 020109 BT
<160> 2
<170> PatentIn version 3.1
<210> 1
   <211> 27
   <212> DNA
   <213> synthetic sequence
<220>
   <221> Primer
   <222> (1)..(27)
   <223> hdeAB1
<400> 1
   gacggctctt tctctagata gttgagg 27
<210> 2
   <211> 25
   <212> DNA
   <213> synthetic sequence
<220>
   <221> Primer
   <222> (1)..(25)
   <223> hdeAB2
<400> 2
   gcaagatggc taagcttgct actcc 25